# FASCICULE DE BREVET EUROPEEN

(11) **EP 0 725 836 B1**
(45) Date de publication et mention de la délivrance du brevet: **13.08.1997**
(21) Numéro de dépôt: 95900165.2
(22) Date de dépôt: 27.10.1994
(51) Int. Cl.: C12Q 1/68, C07H 21/00, C12P 19/34, C12N 1/16

(54) **SONDE ET PROCEDE DE DETECTION DES LEVURES DE L'ESPECE CANDIDA KRUSEI**
SONDE UND VERFAHREN ZUM NACHWEIS VON HEFEN DER ART CANDIDA KRUSEI
PROBE AND METHOD FOR DETECTING YEASTS OF SPECIES CANDIDA KRUSEI

(30) Priorité: 27.10.1993 FR 9313035
(43) Date de publication de la demande: 14.08.1996
(73) Titulaire: BIOMERIEUX SA, F-69280 Marcy L'Etoile (FR)
(72) Inventeur: CARLOTTI, Arnaud, F-69003 Lyon (FR); VILLARD, Jean, F-69170 St. Romain-au-Mont-d'Or (FR)
(74) Mandataire: Fleurance, Raphael
(86) Numéro de dépôt international: FR9401250
(87) Numéro de publication internationale: WO9511991

(56) Documents cités:
- EP-A- 0 335 633
- EP-A- 0 422 869
- WO-A-90/05195
- JOURNAL GENERAL MICROBIOLOGY, vol.138, no.5, Mai 1992, GB pages 901 - 907 WICKES, B. ET AL. 'The molecular analysis of synonymy among medically important yeasts within genus Candida'
- JOURNAL OF CLINICAL MICROBIOLOGY, vol.31, no.8, Août 1993, WASHINGTON US pages 2124 - 2133 SULLIVAN, D. ET AL. 'oligonucleotide fingerprinting of isolates of Candida species other than C. albicans'
- JOURNAL OF CLINICAL MICROBIOLOGY, vol.31, no.4, Avril 1993, WASHINGTON US pages 904 - 910 NIESTERS, H. ET AL 'rapid, polymerase chain reaction-based identification assays for Candida species' cité dans la demande

## Description

### DOMAINE TECHNIQUE :

La présente invention concerne la détection et la caractérisation de levures appartenant à l'espèce *Candida krusei.* En particulier, elle fournit une sonde spécifique, notamment nucléique, pour la détection et, en particulier, l'identification de l'espèce *Candida krusei* et le typage (caractérisation infra-spécifique) des souches de l'espèce *Candida krusei.*

### ART ANTERIEUR :

Les levures sont des champignons, micro-organismes eucaryotes, chez lesquels la forme unicellulaire est prédominante (Barnett J. W., R. W., Payne and D. Yarrow., Yeasts characteristics and identification, 2nd Edition, Cambridge university press, Cambridge, 1991).

Le genre *Candida* est un genre hétérogène, qui regroupe des espèces anamorphes, c'est-à-dire dont on ne connaît ou connaissait pas le mode de reproduction sexuée. Lorsque ce mode de reproduction sexuée est découvert, les espèces sont généralement reclassées au sein d'une espèce (téléomorphe) déjà décrite. Ainsi, *Issatchenkia orientalis* est la téléomorphe de *Candida krusei.*

Il est donc possible de rencontrer, en taxinomie des levures, deux noms d'espèce différents pour décrire un même taxon (groupe) de levures.

En pratique courante, le nom d'espèce *Candida krusei* est le plus usité et c'est celui-ci qui sera employé dans le présent exposé pour désigner indifféremment *Candida krusei* ou *Issatchenkia orientalis.*

*Candida krusei* est l'une des dix principales espèces de levures du genre *Candida* responsables d'infections chez l'homme ou l'animal. Cette espèce est parfois responsable de dégradations de produits alimentaires. Elle est également utilisée pour certaines biotransformations industrielles.

Les infections à *Candida krusei* sont appelées "Candidoses à *C. krusei",* ou "Mycoses à *C.krusei",* elles peuvent concerner pratiquement tous les tissus du corps humain. Les infections systémiques, généralisées ou profondes sont les plus graves et peuvent être fatales. Le taux de mortalité peut être élevé.

Les levures de l'espèce *Candida krusei* sont opportunistes et ubiquistes leur prévalence et leur pathogénicité sont élevées chez les sujets immunodéprimés, notamment chez les patients : neutropéniques, atteints de SIDA, cancéreux, etc, qui sont particulièrement sensibles aux infections à C. *krusei.*

Enfin les candidoses à *C. krusei* sont évidemment très contagieuses à l'instar de toutes les autres affections fongiques.

Toute la difficulté du traitement de ce type d'infections, provient du fait que les souches de *Candida krusei* sont relativement résistantes à certains antifongiques et en particulier au fluconazole. Mais ce qui est encore plus gênant, c'est que l'utilisation de ces antifongiques en prophylaxie, voire en thérapie, favoriserait la colonisation des sujets par des souches endogènes lorsqu'elles sont présentes chez le patient.

En conséquence, une détection rapide et une identification précise et fiable ainsi qu'un typage des souches de cette espèce sont de plus en plus nécessaires, notamment pour être en mesure de mettre en place un traitement antifongique approprié. Il existe, en outre, un réel besoin en marqueurs épidémiologiques fiables, à des fins d'études épidémiologiques ou de traçage des souches dans les industries agro-alimentaire ou de la fermentation.

Mais force est de constater que, les méthodes classiques d'identification des levures ne donnent pas entière satisfaction à tous ces égards.

On connaît ainsi, notamment celles décrites par Barnett J. W., R. W., Payne et D. Yarrow., dans : "Yeasts characteristics and identification", 2nd Edition, Cambridge university press, Cambridge, 1991, qui nécessitent un isolement préalable du micro-organisme en culture pure, puis l'étude de ses caractéristiques morphologiques et surtout physiologiques (plus de 80 tests), l'ensemble de ces opérations requérant de 10 à 30 jours pour une identification. Ces méthodes sont donc fastidieuses et particulièrement longues. Elles sont réservées aux laboratoires de référence et inutilisables en pratique courante.

Plus rapides et plus rationnelles, les méthodes miniaturisées et standardisées d'identification des levures d'importance médicale, comprennent cependant toujours un isolement préalable puis une identification du micro-organisme à l'aide de tests physiologiques, réalisés par exemple à l'aide des galeries. La durée de tels tests est d'environ 5 à 7 jours pour aboutir à une identification. De plus ces techniques ne permettent pas toujours d'identifier l'espèce *Candida krusei* de façon univoque, les confusions avec des espèces telles que *Candida glabrata, Candida inconspicua, Candida lipolytica, Candida norvegensis, Candida rugosa* et *Candida valida* étant possibles voire fréquentes.

Avec les progrès de la biologie moléculaire, une nouvelle technologie utilisant le principe d'hybridation ADN-ADN ou ADN-ARN s'est développée pour la mise au point de tests d'identification rapides, sensibles et spécifiques. Le matériel génétique des micro-organismes présents dans l'échantillon est décelé directement à l'aide de sondes à ADN ou ARN marqué. La détection et l'identification peuvent être réalisées simultanément sans isolement préalable.

Les rares méthodes décrites d'identification de *Candida krusei* à l'aide de sondes nucléiques, présentent l'inconvénient d'être basées sur des sondes de très petite taille (30 à 35 nucléotides) qui nécessitent la mise en oeuvre d'un marquage radioactif plus sensible que le marquage non-radioactif mais aussi plus contraignant.

Un autre inconvénient de ces sondes lié à leur taille réduite, s'exprime dans le cadre de leur mise en oeuvre dans des techniques de polymérisation en chaine (PCR). Ces dernières consistent à amplifier une séquence d'ADN donnée, en la multipliant à partir d'un couple d'amorces double brin et à l'aide d'une polymérase. De telles amplifications peuvent être employées dans un but diagnostique, afin de faciliter la détection. Dès lors que ces sondes seraient utilisées comme amorces en PCR cela limiterait les possibilités de choix des amorces dans une petite séquence de 30 à 35 nucléotides.

Ces sondes sont généralement dirigées contre des séquences cibles d'ADN codant les petites sous unités 5S ou 18 S de l'ARN ribosomique (ssuARNr). Ces cibles sont classiquement choisies car les séquences d'ADN codant les ARN ribosomaux sont les premières à avoir été déterminées. Il existe un problème lié au choix de séquences codant les ssuARNr comme cibles. En effet, ces séquences ont un caractère universel, c'est-à-dire qu'une partie importante d'entre elles est retrouvée chez tous les micro-organismes (levures, champignons bactéries), elles sont dites "hyper-conservées".

En outre, les ssuARNr sont présentes en grande quantité dans les cellules, ce qui a pour conséquence d'augmenter la sensibilité des méthodes d'identification basées sur sa détection.

Il s'ensuit qu'au moins pour ces deux motifs, elles peuvent être à l'origine de faux positifs à la fois en PCR et en détection.

En outre, parmi les sondes décrites dans l'art antérieur, aucune ne permet le typage des souches, c'est-à-dire la différenciation des individus au sein de l'espèce, ces individus étant uniquement assignés à un groupe : l'espèce *Candida krusei.* En effet, les sondes préalablement décrites ne permettent pas un typage des souches de *C. krusei* car elle ne révélent pas suffisamment de polymorphisme de la taille des fragments de restriction parmi les souches de cette espèce *Candida krusei* et ainsi elle ne constituent pas un marqueur épidémiologique fiable.

A titre d'illustrations de telles sondes connues et répondant imparfaitement aux besoins techniques existant en la matière on peut citer :
- les sondes oligonucléotidiques développées par la société Gene-Trak Systems et dirigées contre des séquences de 30 (probe 1351), 33 (probe 1537) et 35 (probe 1530) nucléotides de l'ADN codant l'ARN ribosomal 18S. Ces sondes sont décrites dans la demande de brevet européen n°0422 869. Elles ont été testées contre 4 souches de *Candida krusei.* Il est à noter qu'il n'est pas fait état dans les exemples de cette demande, de détection de *Candida krusei* par hybridation dans les prélèvements de sang humain, crachats ou liquide céphalorachidien. Par ailleurs, ces sondes ne permettent pas de différencier les souches entre elles au sein de l'espèce *Candida krusei,*
- la sonde décrite par Niesters et collaborateurs (Niesters *et al.,* 1993, Rapid, polymerase chain reaction-based identification assays for *Candida* species. J. Clin. microbiol., 31, 904-910) 1993). Il s'agit d'une sonde de 20 nucléotides (oligonucléotide 705) dirigée contre une séquence spécifique du produit d'amplification de la petite sous unité d'ARN ribosomal (ssuARNr). Cette étude est utilisée dans une méthode d'identification des espèces du genre *Candida* basée sur l'amplification en chaine (PCR) de séquences du gène codant la ssuARNr puis le séquençage direct de ces séquences. Cette méthodologie concerne là encore, l'ARN ribosomal. Elle présente l'inconvénient de nécessiter un séquençage direct du produit d'amplification, technique qui n'est accessible qu'à un nombre limité de laboratoires de recherche et surement pas applicable en routine. De plus d'après les auteurs, l'oligonucléotide 705 ne permet pas une amplification spécifique de l'ADN de *Candida krusei.* Les sondes décrites par Niesters et collaborateurs ne permettent pas elles non plus de typer les souches de *Candida krusei.*

Wickes et collaborateurs (Wickes et al., 1992, J. General Microbiology, 138, 901-907) décrivent une sonde CK13 constituée par un fragment d'ADN génomique de taille égale à 0,9 kb, isolée à partir de C. krusei, et presentée comme n'étant pas intraspécifique.

### BREVE DESCRIPTION DE L'INVENTION :

Dans cet état de la technique, l'un des objectifs essentiels de la présente invention est de fournir une sonde de détection des levures de l'espèce *Candida krusei* qui soit parfaitement spécifique.

Un autre objectif de l'invention est de fournir une sonde, notamment nucléique, qui soit de taille suffisante pour autoriser l'utilisation d'un marqueur autre que radioactif et donc de mise en oeuvre plus commode.

Un autre objectif de l'invention est que cette sonde nucléique soit dirigée contre des séquences cibles d'ADN différent de celui codant pour des petites sous unités 5 S ou 18 S d'ARN ribosomique qui sont globalement peu représentatives de l'espèce, car très conservées chez les microorganismes.

Un autre objectif de l'invention est que cette sonde puisse être utilisée dans des milieux complexes biologiques du type sang humain, crachat, ou liquide céphalorachidien.

Un autre objectif de l'invention est que la sonde à *Candida krusei* permette de dépasser la simple détection pour accéder à l'identification précise et au typage des levures au sein même de l'espèce *Candida krusei.*

Ces objectifs, parmi d'autres, sont atteints par la présente invention qui concerne une sonde de détection spécifique et/ou infraspécifiques des levures de l'espèce *Candida krusei* caractérisée en ce qu'elle est choisie parmi les outils génétiques (ou apparentés) suivants :
- au moins une partie :
   * d'au moins un fragment **F** d'acide nucléique ADN et/ou ARN, de taille comprise entre 7 et 4 kb, hybridant spécifiquement avec de l'ADN et/ou de l'ARN de *Candida krusei* et ne codant pas pour la synthèse d'ARN ribosomal,
   * et/ou d'au moins un analogue **Fa** de ce (ou ces) fragment(s) **F** résultant de la dégénérescence du code génétique,
   * et/ou d'au moins un fragment **Fc** d'ADNc complémentaire du fragment **F**,
- au moins une partie des produits de transcription du (ou des) susdit(s) fragment(s) **F** et/ou **Fa** et/ou **Fc**,
- au moins une partie des produits de traduction du (ou des) susdit(s) fragment(s) **F** et/ou **Fa** et/ou **Fc**,
- et une association des outils cités ci-dessus.

### DESCRIPTION DETAILLEE DE L'INVENTION :

Il est donc du mérite de la Demanderesse, d'une part, d'avoir pu isoler au moins un fragment **F** d'acide nucléique de taille importante supérieure ou égale à 4 kb ainsi que l'ARNm et les protéines susceptibles d'en découler et, d'autre part, d'utiliser ces outils génétiques ou apparentés pour la mise en évidence spécifique des levures de l'espèce *Candida krusei,* de même que pour leur caractérisation infraspécifique.

Dans un premier temps, la stratégie de recherche mise au point par la Demanderesse a tout d'abord consisté à sélectionner une souche particulière de *Candida krusei,* à savoir celle ci-après dénommée LMCK 31. Cette souche provient d'un prélèvement broncho-alvéolaire d'un patient hospitalisé atteint d'une affection à *Candida krusei.*

La souche LMCK 31 qui constitue l'un des objets de la présente invention, a été déposée à la collection nationale de culture de microorganismes (CNCM) de l'Institut Pasteur de PARIS, le 22 Octobre 1993 sous la référence I 1372.

Dans un second temps, la Demanderesse a isolé et caractérisé la sonde, notamment nucléique, selon l'invention qui, dans un mode préféré de réalisation, est constituée par au moins une partie d'au moins l'un de deux fragments **F**_{**1**} et **F**_{**2**}, issus de la digestion de l'ADN total de la souche LMCK 31 par l'enzyme de restriction EcoRI. Ces fragments **F**_{**1**}, **F**_{**2**} sont séparés par électrophorèse en gel d'agarose, extraits puis clonés selon les techniques usuelles en biologie moléculaire.

Ils ont été sélectionnés à partir des profils de restriction de l'ADN total de LMCK 31, parmi une multitude d'autres fragments et au terme de nombreuses recherches et expérimentations.

Les deux fragments **F**_{**1**} et **F**_{**2**} sont de préférence présents dans la sonde selon les proportions relatives suivantes **F**_{**1**} / **F**_{**2**} :
99/1 à 1/99, de préférence 80/20 à 20/80 et, plus préférentiellement encore, à hauteur de 50 : 50 parties en poids environ.

Au moins une partie d'au moins l'un des deux fragments **F**_{**1**}, **F**_{**2**} et de préférence les deux en entier, sont utilisés comme sonde selon le principe d'hybridation ADN - ADN ou ADN - ARN. Cette sonde permet la réalisation de tests de détection et d'identification rapide sensible et spécifique de *Candida krusei* et constitue en outre un excellent marqeur de typage des souches de cette espèce, grâce à son aptitude à révéler un polymorphisme de la taille des fragments de restriction de l'ADN total de *Candida krusei.* Cette dernière propriété constitue l'une, parmi d'autres, des originalités de la présente invention.

Il faut considérer que l'hybridation de la sonde nucléique de l'invention avec des séquences cible d'acide nucléique de *C. krusei,* peut se faire à l'aide de la totalité des fragments **F**_{**1**}, **F**_{**2**} visés ci-dessus mais aussi avec seulement une fraction de ces fragments dont la taille reste, de préférence, supérieure ou égale à 0,1 kb.

Selon une modalité particulièrement avantageuse de l'invention, la sonde, dans sa variante nucléique, est caractérisée en ce que chaque fragment **F**_{**1**}, **F**_{**2**} a une taille comprise entre 6,6 et 4,4 kb. De manière plus préférée encore, **F**_{**1**} et **F**_{**2**} ont respectivement une taille d'environ 5,6 et 5,4 kb.

La sonde nucléique selon l'invention est marquée à l'aide de moyens de marquage aptes à révéler l'hybridation. Ces moyens de marquage peuvent être radioactifs ou non. Attendu que cette sonde a une taille relativement grande et que la quantité de marqueurs fixée à la sonde est proportionnelle à la longueur de celle-ci, il est donc possible d'en fixer beaucoup plus sur la sonde selon l'invention (supérieur ou égal à 4 kb) que sur des sondes faites de séquences nucléotidiques de taille plus réduite (inférieure ou égale à 35 bases). Cela permet de pallier à la moindre sensibilité bien connue du marquage non-radioactif par rapport au marquage radioactif.

Or, les marqueurs non radioactifs sont de manipulation aisée et sont accessibles à tous les laboratoires du secteur médical ou agroalimentaire, à l'inverse des marqueurs radioactifs nécessitant des conditions strictes et réglementées de mise en oeuvre, donc d'utilisation strictement limitée aux laboratoires agréés.

Parmi les marqueurs radioactifs classiques, on peut citer le ³²P.

S'agissant des marqueurs non radioactifs, on peut mentionner à titre d'exemples les enzymes fixées telles que la peroxydase.

En raison de sa grande taille, la sonde selon l'invention peut avantageusement être employée comme source d'amorce(s) spécifique(s) utilisables dans des réactions de polymérisation en chaînes (PCR).

Cette (ou ces) amorce(s) constitue(nt) un autre objet de l'invention.

Une autre particularité de ces fragments **F**_{**1**}, **F**_{**2**} est de ne s'apparier qu'avec l'ADN et/ou l'ARN de *Candida krusei* ou de sa forme parfaite à savoir *Issatchenkia orientalis* et non avec celui d'autres levures d'intérêt médical, champignons filamenteux ou bactéries. Cette spécificité est encore améliorée du fait que ces fragments **F**_{**1**}, **F**_{**2**} ne s'hybrident pas avec un ADN cible codant pour l'ARN ribosomal 18 S, contrairement aux sondes nucléiques connues.

Selon une autre caractéristique de l'invention, au moins l'un des fragments **F**_{**1**}, **F**_{**2**} est apte à s'hybrider avec au moins un des fragments issus de la digestion de l'ADN total de la souche du *Candida krusei* LMCK 31 par l'enzyme de restriction HinfI et de taille comprise entre 7,0 et 2,0 kb de préférence entre 3,8 et 2,5 kb.

En fait, la sonde hybride notamment avec au moins l'un des quatre fragments qui sont issus de cette digestion par Hinfi, et dont les tailles respectives sont les suivantes : environ 3,4 ; 3,5 : 3,1 et 2,9 kb.

La sensibilité de cette sonde est parfaitement adaptée à la détection et/ou au typage de *Candida krusei* par hybridation en "dot-blot" ou en "southern-blot".

Cette sensibilité se vérifie dans des essais indirects d'hybridation (méthode des dépôts, dot-blot, southern blot), mais aussi dans des expériences d'hybridation directes in situ. Ces dernières consistent à faire une empreinte sur filtre de colonies en croissance sur un milieu de culture solide et à amener la sonde d'hybridation directement sur le filtre.

Dans ces conditions, la sonde nucléique selon l'invention permet de détecter une colonie de *candida krusei* après 12 à 24 heures de croissance, parmi plus de 30 colonies de différentes espèces, et ce sans purification préalable.

La sonde à *Candida krusei* peut être de nature nucléique, c'est-à-dire constituée d'acides nucléiques du type ADN, ADNc, ARNm issu de la transcription de l'ADN ou leurs variants et analogues.

Mais selon un autre aspect de l'invention, la sonde peut également être constituée des produits de traduction de ce ou ces ADN et/ou ARN ce sont en fait les protéines synthétisables par lecture de l'information codée par l'ARNm.

Il est intéressant de noter que la sonde selon l'invention peut être employée efficacement dans tout milieu complexe, comme peuvent l'être les milieux biologiques : sang, crachat, liquide céphalorachidien. Cela n'interfère nullement sur ses qualités de détection.

La présente invention concerne par ailleurs un procédé de détection spécifique des levures de l'espèce *Candida krusei,* consistant à mettre en oeuvre au moins une sonde nucléique telle que décrite ci-dessus.

Ce procédé s'inscrit dans le cadre des méthodologies connues dans le domaine de la détection et l'identification génétique des micro-organismes.

Selon ce procédé :
- on extrait l'ADN total génomique des souches à étudier,
- on soumet éventuellement cet ADN total à une digestion enzymatique à l'aide d'au moins une enzyme de restriction,
- on dénature l'ADN total éventuellement digéré,
- on met en présence l'ADN total ainsi dénaturé, avec la sonde elle-même préalablement dénaturée et dotée d'au moins un marqueur, de façon à réaliser l'hybridation,
- on élimine l'ADN et la sonde non hybidée,
- et on révèle l'hybridation à l'aide du marqueur.

Il s'agit d'une technique d'hybridation dans laquelle l'ADN cible du micro-organisme à étudier, est soumis à une dénaturation, avec ou sans digestion enzymatique préalable. L'étape qui suit est celle de réassociation des brins séparés d'ADN dénaturé avec la sonde pour reformer des appariements de paires de base originaux.

Lors de cette étape de réassociation, les molécules simple brin de la sonde et de la cible sont mises en condition plus ou moins favorables pour l'appariement (plus ou moins stringentes). Dans des conditions très stringentes seules les molécules dont les séquences sont complémentaires pour un grand nombre de bases, s'hybrident pour former une molécule double brin. La sonde est alors spécifique de la cible.

Avec une sonde marquée à l'aide d'un élément radioactif, tel que le P³², ou d'une enzyme greffée comme par exemple la peroxydase, l'hybridation est aisément révélée qualitativement et quantitativement.

Il ressort de ce qui précède que la sonde nucléique et/ou protéique selon l'invention ainsi que le procédé en faisant application, sont parfaitement spécifiques de *Candida krusei* et offrent une excellente sensibilité, qui leur ouvre des débouchés tout à fait intéressants dans les domaines applicatifs de l'identification et du criblage différenciation des souches de cette espèce (diagnostic médical, contrôles industriels en alimentaire, fermentation...). La détection de *Candida krusei* est obtenue rapidement (24 à 48 heures) et de manière univoque. Ce sont là encore autant d'atouts économiques et industriels pour la sonde et le procédé selon l'invention.

Outre la détection qui regroupe l'identification et le typage de micro-organismes du type *Candida krusei,* la sonde selon l'invention pourrait être appliquée dans le cadre d'une stratégie thérapeutique dirigée à l'encontre des affections à *Candida krusei.* Plus précisément, cela signifie que les fragments formant la sonde peuvent être employés comme cible à atteindre, pour des principes actifs contre les *Candida.* Ce rôle de cible pour médicaments pourrait être tenu par l'ADN ou ses produits de transcription (ARNm) ou de traduction (protéines).

L'invention sera mieux comprise, d'autres de ses avantages et variantes de réalisation ressortiront bien, à la lumière des exemples non limitatifs qui suivent et qui décrivent, en référence aux dessins annexés, la constitution d'une sonde selon l'invention, son obtention et plusieurs procédures de détection de *C. krusei* en faisant application.

### BREVE DESCRIPTION DES FIGURES :

Les dessins annexés comprennent :
- la **fig. 1** qui représente les résultats de l'hybridation de la sonde **F**_{**1**}, **F**_{**2**} avec les fragments de restriction par EcoRI ou HinfI de l'ADN total de Candida krusei LMCK 31 ;
- la **fig. 2** qui représente un profil d'hybridation de la sonde **F**_{**1**}, **F**_{**2**} selon la méthode de Southern avec diverses souches de *Candida krusei ;*
- la **fig. 3a** qui est une photographie d'une séparation par électrophorèse sur gel d'agarose, de fragments d'ADN total obtenu par digestion de différents *Candida* à l'aide d'EcoRI ;
- et la **fig. 3b** qui représente un profil d'hybridation selon la méthode de Southern sous vide des souches de la figure 3a avec la sonde **F**_{**1**}, **F**_{**2**}.

### EXEMPLES

### EXEMPLE 1 : SONDE NUCLÉIQUE F₁, F₂

Dans le présent exemple, la sonde selon l'invention est constituée par deux fragments **F**_{**1**}, **F**_{**2**} issus de la digestion de l'ADN total de la souche LMCK 31 par EcoRI et de taille respective d'environ 5,6 et 5,4 kb.

Les fragments **F**_{**1**}**, F**_{**2**} ont été selectionnés parmi la multitude de fragments produits par la susdite digestion. Ils ont été séparés par électrophorèse en gel d'agarose puis extraits et enfin clonés selon les techniques classiques de biologie moléculaire.

Le matériel de base ayant permis cet isolement, à savoir la souche de *Candida krusei* est LMCK31 a été isolée d'un prélèvement broncho-alvéolaire chez un patient hospitalisé. Cette souche a été déposée le 22 Octobre 1993 à la CNCM de l'Institut Pasteur de PARIS sous la référence I 1372.

### EXEMPLE 2 : HYBRIDATION SELON LA MÉTHODE DES DÉPÔTS DE LA SONDE F₁, F₂ AVEC DES SOUCHES DE LEVURES DE DIVERSES ESPÈCES

L'analyse d'hybridation selon la méthode des dépôt, conformément aux procédures bien connues de l'homme de l'art, nécessite d'immobiliser un acide nucléique ou une population d'acides nucléiques, préalablement dénaturé(s), sur une membrane ou sur un filtre, tel que les membranes en nylon positivement chargées ou les filtres de nitrocellulose ou autre membranes spécialement concues à cet effet, qui peuvent être obtenues facilement dans le commerce. L'ADN ou l'ARN peut être facilement immobilisé sur de telles membranes ou filtres et peut ensuite être sondé ou testé pour hybridation dans de multiples conditions de stringence avec des séquences nucléotidiques ou des sondes considérées. En conditions stringentes, les sondes dont les séquences nucléotidiques possèdent la complémentarité la plus grande avec la cible, montrent un niveau d'hybridation plus important que les sondes dont les séquences présentent moins d'homologies (sont moins complémentaires).

La sonde **F**_{**1**}**, F**_{**2**} de la présente invention est testée par hybridation selon la méthode des dépôts. Ainsi, 6 à 1,5 µg d'ADN total cible des souches à tester et énumérées dans le tableau 1 ci-après, purifié par extraction au phenol sont dénaturés (100 °C, 5 min) et déposés sur une membrane en nylon positivement chargée (Appligène, Illkirch, France) avec l'appareil MilliBlot (Millipore Corporation, Bedford, MA). La membrane est pré-hybridée 2 heures à une température de 41,5 °C en tampon ECL (40 ml), en présence de 5 % (m/v) d'agent bloquant (Amersham, Les Ulis, France), et à une concentration en NaCl de 0,42 M. Une quantité de 600 ng de la sonde **F**_{**1**}, **F**_{**2**} constituée à part égale des deux fragments d'environ 5,6 et 5,4 kb issus de la restriction de l'ADN total de LMCK31 par EcoRI, est marquée à la peroxydase (système ECL) (Amersham, Les Ulis, France). La sonde est ajoutée au milieu réactionnel et l'hybridation de la sonde avec l'ADN cible est menée pendant 6 à 18 heures à une température de 41,5 °C en tampon ECL (40 ml), en présence de 5 % (m/v) d'agent bloquant (Amersham, Les Ulis, France), et à une concentration en NaCl de 0,42 M . La sonde non hybridée est éliminée par deux lavages successifs de 15 min à 41.5 °C avec une solution 1 (urée 6M, Sodium Dodécyl Sulfate 0,4 % (m/v), 20 X SSC 2,5 % (v/v)) durant 5 à 20 min, puis deux lavages successifs à température ambiante avec la solution 2 (2 X SSC) durant 5 à 20 min. La membrane est ensuite immergée pendant 1 min dans un mélange à volume égal des réactifs de révélation 1 et 2 [Système ECL, (Amersham, Les Ulis, France)], puis recouverte par un film photographique Hyperfilm®-ECL pendant 5 à 30 min, selon les recommandations du fabricant. Un signal d'hybridation trés supérieur au bruit de fond est observé uniquement avec l'ADN de *C. krusei* ou de *l. orientalis.*

Dans un essai complémentaire, il a pu être constaté que des quantités de 15 µg d'ADN génomique cible appartenant aux espèces différentes de *Candida krusei* et citées dans le tableau 1, ne permettent pas d'obtenir de signal d'hybridation supérieur à celui du témoin 1,5 µg de *C. krusei.*

### EXEMPLE 3 : HYBRIDATION SELON LA MÉTHODE DE SOUTHERN DE LA SONDE F₁, F₂, AVEC DES FRAGMENTS DE RESTRICTION PAR ECORI OU HINFI DE DIFFÉRENTES SOUCHES DE Candida krusei ET DE DIFFÉRENTES SOUCHES D'ESPÈCES DIVERSES.

Le procédé de transfert selon Southern est une technique essentielle en biologie moléculaire et comporte les étapes suivantes:
- l'ADN total (génomique) des souches à étudier est digéré par une enzyme de restriction et les fragments de restriction qui en résultent sont séparés par électrophorèse selon leur taille sur un gel d'agarose (0,8 % m/v),
- l'ADN séparé est dénaturé dans le gel par une solution de soude puis neutralisé en tampon Tris,
- l'ADN est transféré par aspiration sous vide (système vacugene, Appligene), en haute concentration saline, du gel sur le filtre ou la membrane en nylon positivement chargée,
- l'ADN ainsi transféré est fixé sur le filtre par cuisson à 80 °C pendant 20 min,
- le filtre sur lequel l'ADN est fixé est ensuite pré-hybridé dans un tampon spécial qui sature les sites de liaison non spécifiques par un ADN porteur ou des polymères synthétiques, puis hybridé dans le tampon d'hybridation contenant la sonde dénaturée qui peut être marquée radioactivement (marquage chaud) ou non (marquage froid) par exemple par fixation d'une enzyme telle que la peroxydase.

La température et les conditions d'hybridation sont déterminées pour permettre une hybridation sonde-cible adéquate.

A la suite de l'hybridation la sonde résiduelle non spécifiquement hybridée et fixée sur le filtre est éliminée par une série de lavages. Ceux-ci ne décrochent pas la sonde fixée spécifiquement sur la cible.

La sonde **F**_{**1**}, **F**_{**2**} de la présente invention est testée par hybridation selon la méthode de Southern. 25 à 5 µg d'ADN total cible, purifié par extraction au phenol sont digérés par une enzyme de restriction dans les conditions préconisées par le fabricant (Appligene). Les fragments restreints sont séparés par électrophorèse en gel d'agarose à 0,8 % en tampon Tris-Borate-EDTA (TBE) 1 X, à 2 V/cm pendant 18 h.

Après électrophorèse, les fragments séparés sont transférés selon les techniques classiques de transfert alcalin sur membrane en nylon positivement chargée (Appligene). Après fixation de l'ADN par cuisson de la membrane de transfert à 80 °C pendant 20 min, celle-ci sert de support pour les hybridations.

La membrane est incubée 2h à 41,5 °C dans 40 ml d'une solution de pré-hybridation constituée du tampon ECL (Amersham) contenant 5 % (m/v) d'agent bloquant (Amersham) et 0,42 M NaCl. Les incubations se déroulent en tube dans un four à hybridation à agitation rotative de 20 à 50 rotations par minute (RPM).

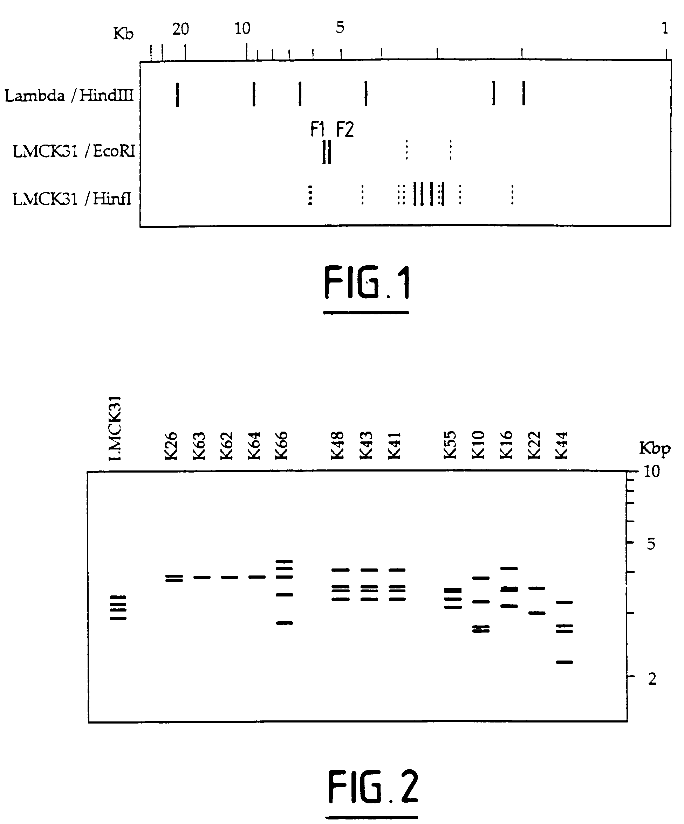

Après la pré-hybridation, 600 ng de la sonde CK1,2 marquée à la peroxydase avec le sytème ECL (Amersham, Les ulis, France) selon les recommandations du fabricant, sont ajoutés au milieu réactionnel. L'hybridation est conduite 18 h à 41,5 °C, avec agitation entre 20 et 50 rpm. La fraction de sonde non hybridée est ensuite éliminée par deux lavages successifs avec la solution 1 [urée 6M, Sodium Dodécyl Sulfate 0,4 % (m/v), 20 X SSC 2,5 % (v/v)] durant 5 à 20 min, puis deux lavages successifs à température ambiante avec la solution 2 (2 X SSC) durant 5 à 20 min. La membrane est ensuite immergée pendant 1 min dans un mélange à volume égal des réactifs de révélation 1 et 2 [Système ECL, (Amersham, Les Ulis, France)], égouttée, enveloppée dans du cello-frais, puis recouverte par un film photographique Hyperfilm®-ECL pendant 5 à 30 min, selon les recommandations du fabricant.
* Dans les essais correspondants au profil d'hybridation de la **fig. 1**, on a mis en oeuvre :
   - un étalon de référence pour le nombre de nucléotides obtenu par restriction à l'aide de HindIII de l'ADN total du phage Lambda ;
   - un premier essai dans lequel la cible est formée par les fragments de restriction par EcoRI de l'ADN total de *Candida krusei* LMCK 31 ;
   - un deuxième essai dans lequel la cible est formée par les fragments de restriction par EcoRI de l'ADN total de Hinfi.

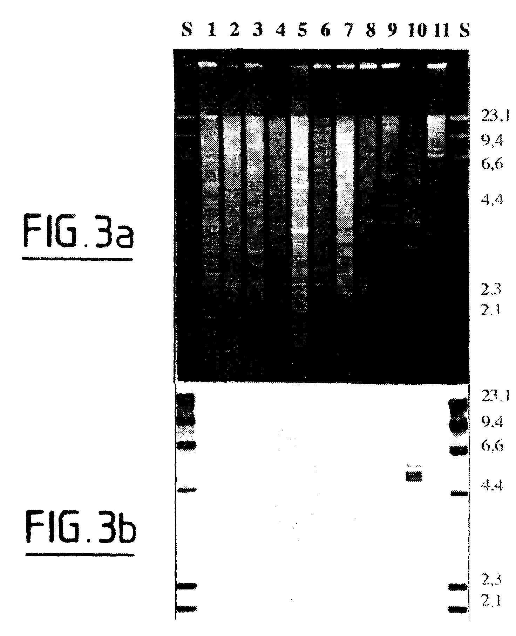

   Sur cette **fig. 1**, les traits pleins correspondent aux fragments hybridant avec **F**_{**1**}, **F**_{**2**}. Les traits pointillés sont les fragments de restriction majeurs facilement observables directement sur les profils de restriction. Les tailles des fragments sont exprimées en kilobases.
* Dans les essais correspondants aux profils d'hybridation de **la fig. 2**, on a utilisé des fragments de restriction par Hinfi de l'ADN total de différentes souches de *Candida krusei :* LMCK 31, K26, K63, K62, K64, K66, K48, K43, K41, K55, K10, K16, K 22 et K44. Les souches K62, K63 et K64 proviennent d'un même patient. Il en va de même pour les souches K41, K43 et K48.
   Chaque bande représente un fragment hybridant avec **F**_{**1**}, **F**_{**2**}.
   Cela montre qu'il est possible d'effectuer un typage des différentes souches au sein de l'espèce *Candida krusei,* à l'aide de l'une des sondes selon l'invention, puisque celle-ci met bien en évidence le polymorphisme de la taille des fragments de restriction de l'ADN de ces souches de *Candida krusei.*
* Dans les essais correspondant aux **fig. 3a** et **3b,** les microorganismes considérés sont les suivants :
   S : Standard : phage lambda digéré par HindIII :
   1 : *Candida tropicalis* 1058 ; 2 : *C. parapsilosis* CBS 604T ; 3 : *C. guillermondii* CBS 6021T ; 4 : *C. lusitaniae* H 278 ; 5 : *C. kefyr ;* 6 : *C. albicans* sérotype B ; 7 : *C. albicans* sérotype A ; 8 : *C. albicans* ATCC 2091 ; 9 : *C. valida* CBS 638T ; 10 : *C*. *krusei* CBS 573T ; 11 : *Yarrowia lipolytica* CBS 6124T [légendes CBS, ATCC (Cf. tableau 1)].

   Dans ces essais, les fragments d'ADN total (6 à 8 µg) digéré par EcoRI des susdits microorganismes, sont séparés par électrophorèse et photographiés (**fig. 3a**), puis transférés sur membrane en nylon selon la méthode de Southern sous vide, et hybridés avec la sonde **F**_{**1**}, **F**_{**2**} dans les conditions décrites ci-dessus. La **fig. 3b** montre le profil d'hybridation obtenu.

### EXEMPLE 4: HYBRIDATION DIRECTE DES COLONIES

Pour le criblage rapide des prélèvements cliniques ou autres, il peut être utile de réaliser des hybridations directement sur les colonies de levures obtenues après étalement du prélèvement sur un milieu de culture adéquat et incubation, par exemple sur milieu de Sabouraud incubé à 28 °C pendant 24h. Ainsi, il est possible d'identifier une colonie de *C. krusei* parmi plusieurs colonies à l'aide de sondes spécifiques.

La sonde **F**_{**1**}**, F**_{**2**} est testée dans les conditions suivantes; les souches de différentes espèces et de *C. krusei* sont ensemencées ponctuellement à la surface d'un milieu de Sabouraud en boîte de Petri, selon les méthodes classiques de la microbiologie. Quinze à trente souches sont étudiées simultanémment. Après 24 h d'incubation entre 30 et 35 °C, une empreinte des colonies est effectuée par application d'un disque de membrane en nylon positivement chargée (membrane positive, Appligene) à la surface du milieu de culture au contact des colonies avec un poids uniforme (environ 10 à 50 grammes). Lorsque le disque est entièrement humidifié, il était retirée de la surface et retourné pour que la surface ayant pris l'empreinte des colonies soit dirigée vers le haut. Le disque est ensuite déposé à la surface d'une feuille de papier Whatman (Whatman 3MM) saturé avec 0,5 M de NaOH (soude).

La membrane est ensuite rincée deux fois par immersion dans 400 ml de 5X SSC avec agitation viguoureuse pour éliminer les débris cellulaires fixés à la surface de la membrane. Le disque est ensuite partiellement séché face supérieure vers le haut sur papier whatman 3MM. Le disque est ensuite utilisé pour les hybridations, dans les conditions identiques à celles décrites précédemment. Le disque de membrane est hybridé dans 40 ml de tampon ECL, 5 % agent bloquant, 0,42 M NaCl avec 400 ng de la sonde **F**_{**1**}, **F**_{**2**} préalablement dénaturée et marquée à la peroxydase avec le système ECL selon les recommandations du fabricant.

Après 18h d'hybridation et les rinçages successifs les seuls signaux d'hybridation sont enregistrés aux emplacements des colonies de *C. krusei et I. orientalis* sur l'empreinte réalisée sur la membrane.

La sonde **F**_{**1**}, **F**_{**2**} peut être également utilisée dans d'autres conditions d'hybridation selon les méthodes classiques de la biologie moléculaire bien connues de l'homme de l'art.

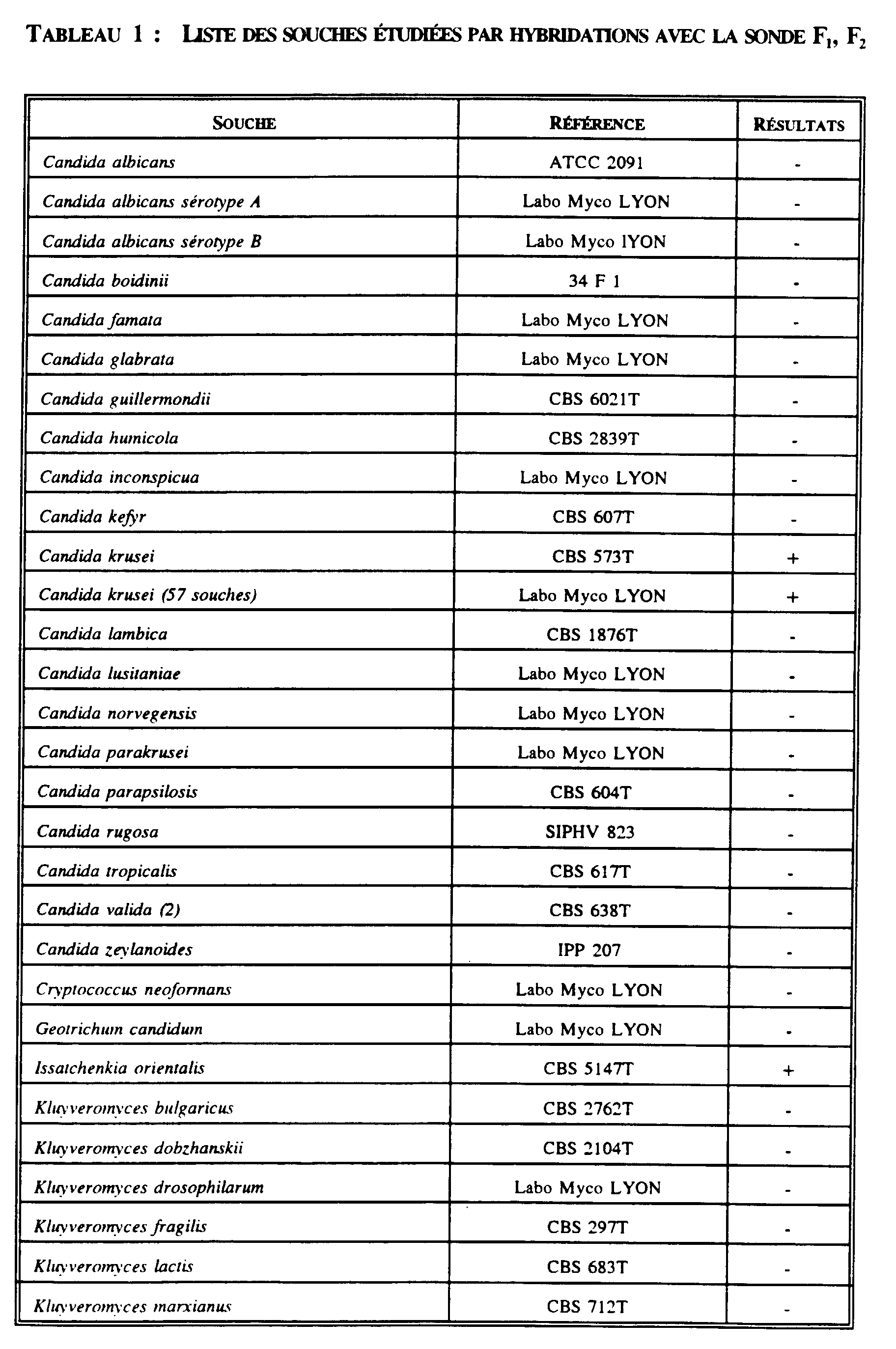

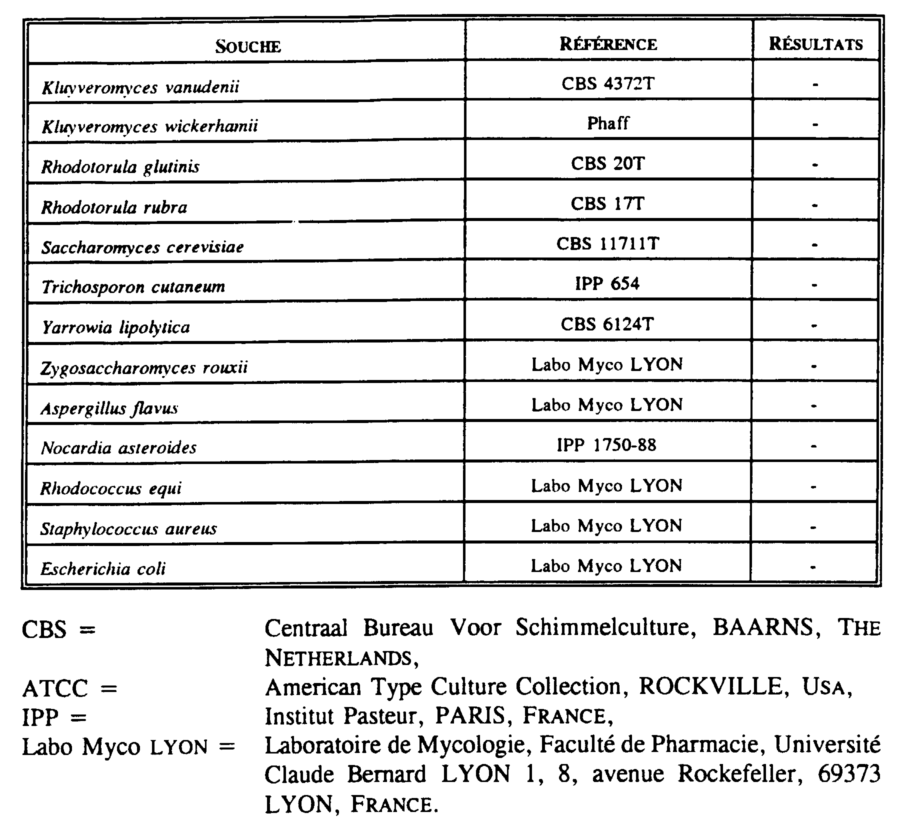

## Revendications

1. Sonde de détection spécifique et/ou infraspécifique de levures de l'espèce *Candida krusei* caractérisée en ce qu'elle est choisie parmi les outils génétiques (ou apparentés) suivants :
- au moins une partie :
* d'au moins un fragment **F** d'acide nucléique ADN et/ou ARN, de taille comprise entre 7 et 4 kb, hybridant spécifiquement avec de l'ADN et/ou de l'ARN de *Candida krusei* et ne codant pas pour la synthèse d'ARN ribosomal,
* et/ou d'au moins un analogue **Fa** de ce (ou ces) fragment(s) **F** résultant de la dégénerescence du code génétique,
* et/ou d'au moins un fragment **Fc** d'ADNc complémentaire du fragment **F**,
- au moins une partie des produits de transcription du (ou des) susdit(s) fragment(s) **F** et/ou **Fa** et/ou **Fc**,
- au moins une partie des produits de traduction du (ou des) susdit(s) fragment(s) **F** et/ou **Fa** et/ou **Fc**,
- et une association des outils cités ci-dessus.

2. Sonde selon la revendication 1, caractérisée en ce qu'elle est constituée par au moins une partie d'au moins l'un des deux fragments **F**_{**1**} et **F**_{**2**} issus de la digestion de l'ADN total de la souche LMCK 31 par l'enzyme de restriction EcoRI.

3. Sonde selon la revendication 2, caractérisée en ce que **F**_{**1**} et **F**_{**2**} ont chacun une taille comprise entre 6,6 et 4,4 kb et de préférence de l'ordre de 5,6 et 5,4 kb respectivement.

4. Sonde selon la revendication 2 ou la revendication 3, caractérisé en ce qu'au moins l'un des fragments **F**_{**1**}, **F**_{**2**} est apte à s'hybrider avec au moins l'un des fragments issus de la digestion de l'ADN total de la souche de *Candida krusei* LMCK 31 par l'enzyme de restriction Hinfi et de taille comprise entre 7,0 et 2,0 kb de préférence entre 3,8 et 2,5 kb.

5. Sonde selon l'une quelconque des revendications 2 à 4, caractérisée en ce qu'elle comporte deux fragments **F**_{**1**} et **F**_{**2**}, présents dans les proportions relatives suivantes **F**_{**1**}**/F**_{**2**} :
99/1 à 1/99, de préférence 80/20 à 20/80 et, plus préférentiellement encore, à hauteur de 50 : 50 parties en poids environ.

6. Sonde selon l'une quelconque des revendications 1 à 5, caractérisée en ce qu'elle est pourvue d'au moins un marqueur.

7. Sonde selon l'une quelconque des revendications 2 à 6, caractérisée en ce que la souche de *Candida krusei* LMCK 31 est celle déposée à la CNCM de l'institut Pasteur le 22 Octobre 1993 sous le numéro I 1372.

8. Procédé de détection spécifique des levures de l'espèce *Candida krusei,* caractérisé en ce qu'il consiste à mettre en oeuvre au moins une sonde selon l'une quelconque des revendications 1 à 7.

9. Procédé selon la revendication 8, caractérisé en ce que :
- on extrait l'ADN total génomique des souches à étudier,
- on soumet éventuellement cet ADN total à une digestion enzymatique à l'aide d'au moins une enzyme de restriction,
- on dénature l'ADN total éventuellement digéré,
- on met en présence l'ADN total ainsi dénaturé, avec la sonde elle-même préalablement dénaturée et dotée d'au moins un marqueur, de façon à réaliser l'hybridation,
- on élimine l'ADN et la sonde non hybridée,
- et on révèle l'hybridation à l'aide du marqueur.

10. Amorce oligonucléotidique, utilisable notamment pour la réaction de polymérisation en chaîne (PCR), caractérisée en ce qu'elle est constituée par la sonde selon l'une quelconque des revendications 1 à 7.

11. Application de la sonde selon l'une quelconque des revendications 1 à 7, pour l'identification des levures de l'espèce *Candida krusei.*

12. Application de la sonde selon l'une quelconque des revendications 1 à 7, pour la différenciation des souches au sein de l'espèce *Candida krusei.*

13. Application de la sonde selon l'une quelconque des revendications 1 à 7 dans le cadre d'une stratégie thérapeutique visant des affections à *Candida krusei.*

14. Souche utile notamment pour l'obtention de la sonde selon l'une quelconque des revendications 1 à 7, caractérisée en ce qu'elle est constituée par la souche *Candida krusei* LMCK 31 déposée à la CNCM le 22/10/93 sous le numéro I 1372.

## Patentansprüche

1. Sonde für den spezifischen und/oder subspezifischen Nachweis von Hefen der Art *Candida krusei,* dadurch gekennzeichnet, daß sie unter den folgenden (oder verwandten) genetischen Werkzeugen ausgewählt ist:
- mindestens einem Teil:
* mindestens eines Fragments F von DNA- und/oder RNA-Nucleinsäure mit einer Größe von 7 bis 4 kb, das spezifisch mit der DNA und/oder der RNA von *Candida krusei* hybridisiert und das nicht die Synthese ribosomaler RNA codiert,
* und/oder mindestens eines analogen Fragments Fa dieses (oder dieser) Fragments (Fragmente) F, das aus der Degenerierung des genetischen Codes resultiert,
* und/oder mindestens eines Fragments Fc der komplementären cDNA des Fragments F,
- mindestens einem Teil der Transkriptionsprodukte des (oder der) obengenannten Fragments (Fragmente) F und/oder Fa und/oder Fc,
- mindestens einem Teil des Produktes der Translation des (oder der) obengenannten Fragmente F und/oder Fa und/oder Fc,
- und einer Kombination der obengenannten Werkzeuge.

2. Sonde nach Anspruch 1, dadurch gekennzeichnet, daß sie aus mindestens einem Teil mindestens eines der beiden Fragmente F₁ und F₂ besteht, die durch Spaltung der gesamten DNA des Stamms LMCK 31 mit dem Restriktionsenzym E*co*RI entstehen.

3. Sonde nach Anspruch 2, dadurch gekennzeichnet, daß F₁ und F₂ jeweils eine Größe von 6,6 bis 4,4 kb und vorzugsweise jeweils eine Größe in der Größenordnung von 5,6 und 5,4 kb aufweisen.

4. Sonde nach Anspruch 2 oder Anspruch 3, dadurch gekennzeichnet, daß mindestens eines der Fragmente F₁, F₂ befähigt ist, mit mindestens einem der Fragmente zu hybridisieren, die durch Spaltung der gesamten DNA des Stamms LMCK 31 von *Candida krusei* mit dem Restriktionsenzym *Hinf*I entstehen und eine Größe von 7,0 bis 2,0 kb und vorzugsweise 3,8 bis 2,5 kb aufweisen.

5. Sonde nach einem der Ansprüche 2 bis 4, dadurch gekennzeichnet, daß sie zwei Fragmente F₁ und F₂ enthält, die in den folgenden relativen Anteilen F₁/F₂ enthalten sind:
99/1 bis 1/99, vorzugsweise 80/20 bis 20/80 und noch bevorzugter im Bereich von etwa 50:50 Gewichtsteilen.

6. Sonde nach einem der Ansprüche 1 bis 5, dadurch gekennzeichnet, daß sie mit mindestens einem Marker versehen ist.

7. Sonde nach einem der Ansprüche 2 bis 6, dadurch gekennzeichnet, daß der Stamm LMCK 31 von *Candida krusei* der Stamm ist, der bei der CNCM-Sammlung des Pasteur-Instituts am 22. Oktober 1993 unter der Nr. I 1372 hinterlegt worden ist.

8. Verfahren für den spezifischen Nachweis von Hefen der Art *Candida krusei,* dadurch gekennzeichnet, daß es darin besteht, mindestens eine Sonde nach einem der Ansprüche 1 bis 7 einzusetzen.

9. Verfahren nach Anspruch 8, dadurch gekennzeichnet, daß es folgende Schritte umfaßt:
- Extrahieren oder Gewinnen der gesamten genomischen DNA
des zu untersuchenden Stamms,
- ggf. Ausführen einer enzymatischen Spaltung dieser gesamten DNA mit Hilfe mindestens eines Restriktionsenzyms,
- Denaturieren der ggf. gespaltenen gesamten DNA
- Zusammenbringen der so denaturierten gesamten DNA mit der Sonde, die selbst zuvor denaturiert und mit mindestens einem Marker versehen worden ist, um die Hybridisierung durchzuführen,
- Entfernen von nicht hybridisierter DNA und Sonde,
- und Feststellen bzw. Nachweisen der Hybridisierung mit Hilfe des Markers.

10. Starter-Oligonucleotid, das insbesondere für die Polymerase-Kettenreaktion (PCR) verwendbar ist, dadurch gekennzeichnet, daß es aus der Sonde nach einem der Ansprüche 1 bis 7 besteht.

11. Verwendung der Sonde nach einem der Ansprüche 1 bis 7 für die Identifizierung von Hefen der Art *Candida krusei.*

12. Verwendung der Sonde nach einem der Ansprüche 1 bis 7 für die Unterscheidung von Stämmen der Art *Candida krusei.*

13. Verwendung der Sonde nach einem der Ansprüche 1 bis 7 im Rahmen einer therapeutischen Strategie, die auf durch *Candida krusei* hervorgerufene Erkrankungen ausgerichtet ist.

14. Stamm, der insbesondere für die Herstellung der Sonde nach einem der Ansprüche 1 bis 7 verwendbar ist, dadurch gekennzeichnet, daß er aus dem Stamm LMCK 31 von *Candida krusei* besteht, der am 22.10.93 bei der CNCM-Sammlung unter der Nummer I 1372 hinterlegt worden ist.

## Claims

1. Probe for the specific and/or infraspecific detection of yeasts of the *Candida krusei* species, characterised in that it is selected from the following genetic (or related) tools :
- at least one part :
* of at least one fragment F of DNA and/or RNA nucleic acid, of size between 7 and 4 kb, which specifically hybridises with DNA and/or RNA of *Candida krusei* and which does not encode the synthesis of ribosomal RNA,
* and/or of at least one analogue Fa of this (or these) fragment(s) F which result(s) from the degeneration of the genetic code,
* and/or of at least one complementary cDNA fragment Fc of the fragment F,
- at least one part of the products of transcription of the above-mentioned fragment(s) F and/or Fa and/or Fc,
- at least one part of the products of translation of the above-mentioned fragment(s) F and/or Fa and/or Fc,
- and one association of the tools cited above.

2. Probe according to claim 1, characterised in that it is constituted by at least one part of at least one of the two fragments F₁ and F₂ resulting from the digestion of the total DNA of the strain LMCK 31 by the restriction enzyme EcoRI.

3. Probe according to claim 2, characterised in that F₁ and F₂ are both of a size between 6.6 and 4.4 kb and preferably in the order of 5.6 and 5.4 kb respectively.

4. Probe according to claim 2 or 3, characterised in that at least one of the fragments F₁, F₂ is fit to be hybridised with at least one of the fragments resulting from the digestion of the total DNA of the *Candida krusei* strain LMCK 31 by the restriction enzyme HinfI, and of size between 7.0 and 2.0 kb, preferably between 3.8 and 2.5 kb.

5. Probe according to any one of claims 2 to 4, characterised in that it comprises two fractions F₁ and F₂ which are present in the following relative proportions F₁/F₂:
99/1 to 1/99, preferably 80/20 to 20/80 and, even more preferentially, up to about 50/50 parts by weight.

6. Probe according to any one of claims 1 to 5, characterised in that it is provided with at least one marker.

7. Probe according to any one of claims 2 to 6, characterised in that the *Candida krusei* strain LMCK 31 is that filed at the CNCM of the Institut Pasteur on the 22 October 1993 under the number I 1372.

8. Method of specific detection of yeasts of the species *Candida krusei,* characterised in that it consists in using at least one probe according to any one of claims 1 to 7.

9. Method according to claim 8, characterised in that :
- the genomic total DNA of the strains to be studied are extracted,
- this total DNA is optionally submitted to an enzymatic digestion with the aid of at least one restriction enzyme,
- the optionally digested total DNA is denatured,
- the total DNA thus denatured is placed in the presence with the probe which is itself beforehand denatured and endowed with at least one marker so as to carry out the hybridisation,
- the DNA and the non-hybridised probe are removed,
- and the hybridisation is visualised with the aid of the marker.

10. Oligonucleotide primer which can be used notably for the polymerisation chain reaction (PCR), characterised in that it is constituted by the probe according to any one of claims 1 to 7.

11. Application of the probe according to any one of claims 1 to 7 for the identification of yeasts of the species *Candida krusei.*

12. Application of the probe according to any one of claims 1 to 7 for the differentiation of the strains within the *Candida krusei* species.

13. Application of the probe according to any one of claims 1 to 7 in the context of a therapeutic strategy against *Candida krusei* diseases.

14. Strain notably useful for obtaining the probe according to any one of claims 1 to 7, characterised in that it is constituted by the *Candida krusei* strain LMCK 31 filed at the CNCM on the 22/10/93 under the number I 1372.
